# EUROPEAN PATENT APPLICATION

(11) **EP 1 862 536 A1**
(43) Date of publication of application: **05.12.2007**
(21) Application number: 06729797.8
(22) Date of filing: 23.03.2006
(51) Int. Cl.: C12N 1/21, C12N 15/09, C12P 7/62

(54) **MICROORGANISM CAPABLE OF ACCUMULATING ULTRA HIGH MOLECULAR WEIGHT POLYESTER**

(30) Priority: 24.03.2005 JP 2005086447
(71) Applicant: The Procter and Gamble Company, Cincinnati, Ohio 45202 (US); Kaneka Corporation, Kita-ku Osaka-shi, Osaka 530-8288 (JP)
(72) Inventor: MARUYAMA, Hiroyuki, go, 6750051 (JP)
(74) Representative: Vossius & Partner
(86) International application number: PCT/JP2006/305849
(87) International publication number: WO 2006/101176

(57) **Abstract**

An object of the present invention is to provide a microorganism strain capable of accumulating P(3HB-co-3HH) having an ultra high molecular weight at a high level, and to provide a method of producing a safe and inexpensive copolymerized polyester using the same. The present invention is related to a microorganism which is obtained by introducing a polyhydroxyalkanoic acid synthase gene and a 3-ketoacyl-ACP reductase gene into at least one host microorganism selected from the group consisting of those belonging to genus *Ralstonia,* genus *Aeromonas,* genus *Alcaligenes* and genus *Pseudomonas* to allow for transformation.

## Description

### Technical Field

The present invention relates to a method of producing a biodegradable polyester having an ultra high molecular weight that exhibits excellent properties in processing characteristics. More specifically, the present invention relates to a method of producing a biodegradable polyester having an ultra high molecular weight using a microorganism in which a polyhydraxyalkanoic acid synthase gene and a 3-ketoacyl-ACP reductase gene are expressed.

### Background Art

Polyhydroxyalkanoic acid (PHA) is a polyester type organic molecular polymer produced by a wide variety of microorganisms. Utilization of these polymers in various industries has been attempted through industrial production as environment-conscious materials or biocompatible materials since they have a biodegradability, are thermoplastic macro molecules, and they can be produced from renewable resources. Monomer unit constituting this polyester has a common name of 3-hydroxyalkanoic acid, and specifically, 3-hydroxybutyric acid, 3-hydroxyvaleric acid, 3-hydroxyhexanoic acid, 3-hydroxyoctanoic acid, or 3-hydroxyalkanoic acid having a longer alkyl chain forms a polymer molecule by homopolymerization or copolymerization.

Although poly-3-hydroxybutyric acid (hereinafter, abbreviated as P(3HB)) that is a homopolymer of 3-hydroxybutyric acid (hereinafter, abbreviated as 3HB) was first found in 1925 in *Bacillus megaterium,* this P(3HB) has high crystallinity, and hard and fragile properties, whereby the range of practical applications thereof is restricted.
In addition, copolymers of 3-hydroxybutyric acid (3HB) and 3-hydroxyvaleric acid (3HV) (hereinafter, abbreviated as P(3HB-co-3HV)) have greater flexibility as compared with P(3HB), and a method of their production was also disclosed (for example, see Patent Document 1, Patent Document 2). However, in fact, even though the 3HV molar fraction is increased in P(3HB-co-3HV), the physical properties accompanied thereby are poorly altered. In particular, the flexibility is not improved, leading to utilization only in fields of hard molded products such as shampoo bottles, disposable handles for razors, and the like.

In addition, middle chain PHA composed of 3-hydroxyalkanoic acid the alkyl chain of which having 6 to 16 carbon atoms has lower crystallinity than P(3HB) or P(3HB-co-3HV), and is highly elastic (see, Nonpatent Document 1), therefore, applications in different fields have been expected. Production and investigation of middle chain PHA have been performed by introducing a PHA synthase gene of a microorganism belonging to genus *Pseudomonas* into a microorganism belonging to genus *Pseudomonas,* genus *Ralstonia,* or *Escherichia coli.* However, the productivity was low in every case, therefore, this process is not suited for industrial production (see, Nonpatent Document 2, Nonpatent Document 3, Nonpatent Document 4).

Moreover, as one type of polyhydroxyalkanoic acid, binary copolymerized polyester (hereinafter, abbreviated as P(3HB-co-3HH)) of 3HB and 3-hydroxyhexanoic acid (hereinafter, abbreviated as 3HH) has been known, and a method of producing the same is disclosed (see, Patent Document 3, Patent Document 4). Furthermore, in Nonpatent Document 5, it is reported that when P(3HB-co-3HH) having a 3HH composition of 11 to 19% by mole was produced by fermentation, a shift of its properties from hard and fragile to flexible was gradually exhibited as the 3HH composition increased, and flexibility greater than that of P(3HB-co-3HV) was finally exhibited. In other words, P(3HB-co-3HH) has a wide range of physical properties permitting applicability of from the hard polyester to the soft polyester by changing the 3HH composition, therefore, applications in a wide range of fields from those requiring hardness such as housing of TV sets to those requiring flexibility such as films and the like can be expected.

The polyester has varying characteristics depending on the molecular weight, and it is desired to have a molecular weight as high as possible in processing into fibers and the like. Although the molecular weight of the polyhydroxyalkanoic acid produced by a microorganism varies according to each microorganism, it is approximately from 50,000 Dalton to 1,000,000 Dalton. Therefore, production of PHA having higher molecular weight was investigated.
A method of producing an ultra high molecular weight P(3HB) having a weight average molecular weight of beyond 10,000,000 by adjusting the pH and glucose concentration in culture using *Escherichia coli* in which a gene was incorporated relating to PHA synthesis derived from *Ralstonia eutropha* was demonstrated. Therein, it is revealed that physical properties (for example, tensile strength and redistractibility) that are critical in processing into fiber and the like are improved with the ultra high molecular weight P(3HB) (see, Nonpatent Document 6, Nonpatent Document 7, Nonpatent Document 8).
Additionally, it was also proven that P(3HB) having a weight average molecular weight of 3,000,000 to 12,000,000 Dalton could be produced by altering the concentration of the PHA synthase in production of P(3HB) *in vitro* (see, Nonpatent Document 9).

Furthermore, it is disclosed that in production of P(3HB) in *Escherichia coli* using an expression vector having a PHA synthase gene or the like controlled by an inducible promoter, the weight average molecular weight could be adjusted to be 780,000 to 4,000,000 Dalton as a result of regulation of the amount of the enzyme expressed depending on the amount of the inducer (see, Patent Document 5).
Moreover, in Patent Document 6, it is disclosed that when a PHA synthase gene was incorporated into and expressed in a bacterial chromosome, PHA having varying molecular weights of from 400,000 to 1,000,000 Dalton could be produced depending on the site of the incorporation. In the case in which a PHA synthase gene derived from *Aeromonas caviae,* and a gene for supplying a substrate monomer were incorporated into a chromosome of *Ralstonia eutropha,* copolymerized polyester of 3-hydroxyhexanoic acid and 3-hydroxyoctanoic acid having a molecular weight of from 400,000 to 10,000,000 Dalton was accumulated. However, its productivity was not referred to.

Moreover, many reports on investigation of production of P(3HB-co-3HH) are also found. In the case of culture using *Aeromonas hydrophila,* P(3HB-co-3HH) was produced with the produced biomass of 95.7 g/L, the polyester content of 45.2% and the 3HH composition of 17%, in feeding culture for 43 hrs using oleic acid as a carbon source (see, Nonpatent Document 10). Additionally, *Aeromonas hydrophila* was cultured using glucose and lauric acid as a carbon source, whereby the produced biomass of 50 g/L and the polyester content of 50% were achieved, with the molecular weight being 1,000,000 Dalton (see, Nonpatent Document 11). However, since *Aeromonas hydrophila* has pathogenicity against human (see, Nonpatent Document 12), it cannot be recognized as a species suited for industrial production. In addition, since expensive carbon sources are used in such production by culture, use of inexpensive carbon sources has been also demanded in light of the production cost.

In an investigation of production of P(3HB-co-3HH) using a safe host, a transformant was used which was obtained by introducing a PHA synthase gene cloned from *Aeromonas caviae* into *Ralstonia eutropha.* The produced biomass was 4 g/L, and the polyester content was 30% (see, Patent Document 7, Nonpatent Document 13). Further, as a result of culture of this transformant using vegetable oil and fat as a carbon source, the produced biomass of 4 g/L, and the polyester content of 80% were achieved, with the weight average molecular weight being 510,000 Dalton (see, Nonpatent Document 14). Also, in the case of a transformant obtained using a promoter of a phb operon of *Ralstonia eutropha* as the promoter, it was demonstrated that P(3HB-co-3HH) with the polyester content of 56% and the weight average molecular weight of 3,100,000 was accumulated in the culture with use of octanoic acid as single carbon source (see, Nonpatent Document 15). However, no report has been found with regard to production of P(3HB-co-3HH) having an ultra high molecular weight in culture using an inexpensive vegetable oil and fat or the like as a carbon source.

On the other hand, aiming at improvement of productivity of P(3HB-co-3HH) and regulation of the 3HH composition, artificial modification of a PHA synthase was carried out. Among the mutants of the PHA synthase derived from *Aeromonas caviae,* a mutated enzyme in which the amino acid asparagine at position 149 is substituted with serine, and a mutated enzyme in which the amino acid aspartic acid at position 171 is substituted with glycine were proven to have improved PHA synthase activity and 3HH composition in *Escherichia coli.* However, the molecular weight was lowered as compared with 2,600,000 Dalton of naturally occurring enzyme (see, Nonpatent Document 16). Moreover, it was reported that the mutant of this enzyme selected on the basis of enhancement of staining with Nile Red as a marker exhibited improved PHA synthase activity in *Escherichia coli,* and the average molecular weight was also elevated to maximum of 358,000 Dalton, i.e., 1.8 times (see, Nonpatent Document 17).

On another hand, attempts to improve productivity of PHA by introducing a fatty acid synthetic enzyme gene together with the PHA synthase gene into a host microorganism were reported (see, Patent Document 8, Nonpatent Document 18). In these reports, productivity of P(3HB-co-3HH) and P(3HB) was successfully improved by introducing a 3-ketoacyl-ACP reductase gene or any of fatty acid synthetic enzyme genes derived from *Escherichia coli* into *Escherichia coli* together with the PHA synthase gene derived from *Aeromonas caviae,* however, the molecular weight of the produced polyester was not referred to.
As set forth hereinabove, according to the prior art of the production of a high molecular weight P(3HB-co-3HH), P(3HB-co-3HH) having a weight average molecular weight of at most 3,100,000 Dalton approximately could be merely produced, and low productivity and use of an expensive carbon source were problematic.
Patent Document 1: Japanese Unexamined Patent Application Publication No. Sho 57-150393;
Patent Document 2: Japanese Unexamined Patent Application Publication No. Sho 59-220192;
Patent Document 3: Japanese Unexamined Patent Application Publication No. Hei 5-93049;
Patent Document 4: Japanese Unexamined Patent Application Publication No. Hei 7-265065;
Patent Document 5: U. S. Patent No. 5811272
Patent Document 6: U. S. Patent No. 6593116
Patent Document 7: Japanese Unexamined Patent Application Publication No. Hei 10-108682;
Patent Document 8: Japanese Unexamined Patent Application Publication No. 2000-135083;
Nonpatent Document 1: Madison et al., Microbiol. Mol. Biol. Rev., 63: 21-53 (1999)
Nonpatent Document 2: Matsusaki et al., J. Bacteriol., 180: 6459-6467 (1998)
Nonpatent Document 3: Matsusaki et al., Appl. Micrbiol. Biotechnol., 53: 401-409 (2000)
Nonpatent Document 4: Langenbach et al., FEMS Microbial. Lett., 150: 303-309 (1997)
Nonpatent Document 5: Doi et al., Macromolecules, 28: 4822-4828 (1995)
Nonpatent Document 6: Kusaka et al., Appl. Micrbiol. Biotechnol., 47: 140-143 (1997)
Nonpatent Document 7: Kusaka et al., J. Macromol. Sci., Pure Appl. Chem., A35: 319-335 (1998)
Nonpatent Document 8: Kusaka et al., Int. J. Biol. Macromol., 25: 87-94 (1999)
Nonpatent Document 9: Gerngross et al., Proc. Natl. Acad. Sci. USA, 92: 6279-6283 (1995)
Nonpatent Document 10: Lee et al., Biotechnol. Bioeng., 67: 240-244 (2000)
Nonpatent Document 11: Chen et al., Appl. Microbiol. Biotechnol., 57: 50-55 (2001)
Nonpatent Document 12: National Institute of Infectious Diseases, Safety Administrative Provisions of Pathogens and the like, Table 1 with Appendix Table (1999)
Nonpatent Document 13: Fukui et al., J. Bacteriol., 179: 4821-4830 (1997)
Nonpatent Document 14: Fukui et al., Appl, Microbiol. Biotecnol., 49: 333-336 (1998)
Nonpatent Document 15: Kichise et al., Int. J. Biol. Macromol., 25: 69-77 (1999)
Nonpatent Document 16: Kichise et al., Appl. Environ, Microbiol., 68: 2411-2419 (2002)
Nonpatent Document 17: Amara et al., Appl. Microbiol. Biotechnol., 59; 477-482 (2002)
Nonpatent Document 18: Taguchi et al., FEMS Microbiol. Lett., 176: 183-190 (1999)

### DISCLOSURE OF THE INVENTION

### Problems that the Invention is to Solve

In view of the foregoing circumstances, an object of the present invention is to provide a microorganism strain capable of accumulating P(3HB-co-3HH) having an ultra high molecular weight at a high level, and to provide a method of producing a safe and inexpensive copolymerized polyester using the same.

### Means for Solving the Problems

The present inventor elaborately investigated in order to solve the aforementioned problems, and consequently found that a transformed microorganism into which a PHA synthase gene (phaC) and a 3-ketoacyl-ACP reductase gene were introduced can accumulate an ultra high molecular weight P(3HB-co-3HH) having a weight average molecular weight of equal to or greater than 3,100,000 Dalton at a high level. The upper limit of the molecular weight of P(3HB-co-3HH) which can be accumulated by the present transformed microorganism is 10,000,000 Dalton, and more preferably 7,000,000 Dalton taking into consideration of industrial production possibility.
As demonstrated in Examples, the transformed strain obtained by introducing a PHA synthase mutant gene (D171G mutant gene) derived from *Aeromonas caviae,* and a 3-ketoacyl-ACP reductase gene *(fabG)* derived from *Escherichia coli* into *Ralstonia eutropha* produced P(3HB-co-3HH) having a weight average molecular weight of 5,100,000 Dalton, with a produced biomass of 109.2 g/L, and a polyester content of 68,6% by culture for 64 hrs.

Accordingly, aspects of the present invention are as in the followings.
[1] A microorganism which is obtained by introducing a polyhydroxyalkanoic acid synthase gene and a 3-ketoacyl-ACP reductase gene into at least one host microorganism selected from the group consisting of those belonging to genus *Ralstonia,* genus *Aeromonas,* genus *Alcaligenes* and genus *Pseudomonas* to allow for transformation, and which is capable of accumulating a copolymerized polyester composed of monomer units of 3-hydroxybutyric acid and 3-hydroxyhexanoic acid.
[2] The microorganism according to the item [1] wherein the host microorganism is *Ralstonia eutropha.*
[3] The microorganism according to the item [1] or [2] wherein the 3-ketoacyl-ACP reductase gene is a gene derived from *Escherichia coli*
[4] The microorganism according to any one of the items [1] to [3] wherein the polyhydroxyalkanoic acid synthase gene is a gene encoding an enzyme derived from *Aeramanas caviae,* or a mutant thereof.
[5] The microorganism according to the item [4] wherein the mutant comprises at least one amino acid substitution of either one of the following (a) or (b):
   (a) substitution of the amino acid asparagine at position 149 with serine; or
   (b) substitution of the amino acid aspartic acid at position 171 with glycine.
[6] The microorganism according to any one of the items [1] to [3] wherein the polyhydroxyalkanoic acid synthase gene encodes a mutant enzyme derived from *Aeromonas caviae* comprising substitution of the amino acid aspartic acid at position 171 with glycine.
[7] A method of producing a polyester using the microorganism according to any one of the items [1] to [6].

Hereinafter, the present invention will be explained in detail.
The copolymerized polyester (P(3HB-co-3HH)) composed of the monomer units of 3-hydroxybutyric acid (3HB) and 3-hydroxyhexanoic acid (3HH) according to the present invention is a copolymerized polymer represented by the following general formula:

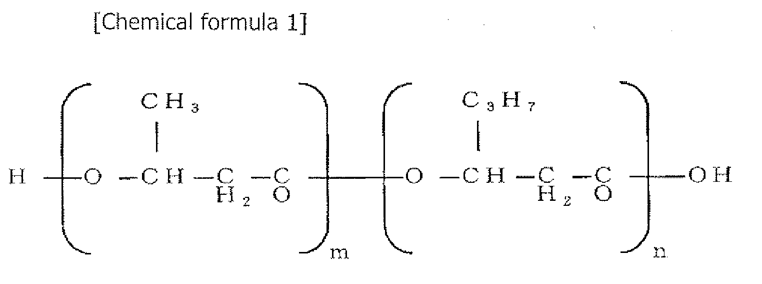

### (wherein, m and n represent the number of the monomer units of the polymer, being 1 or more.)

P(3HB-co-3HH) obtained by the microorganism of the present invention has a weight average molecular weight (Mw) of preferably 3,100,000 to 10,000,000, and more preferably 4,200,000 to 7,000,000. The weight average molecular weight (Mw) is a value determined by a gel permeation chromatographic process.

The host microorganism for use in the present invention is not particularly limited, and any microorganisms isolated from natural resources, microorganisms deposited at a depository organization of microorganism strains (for example, IFO, ATCC and the like), and the like can be used.
Specifically, polyester-nonproducing microorganisms of bacteria belonging to genus *Ralstonia,* genus *Aeromonas,* genus *Alcaligenes,* genus *Pseudomonas* can be used. In view of safety and productivity, the host microorganism belongs to preferably genus *Ralstonia,* and is more preferably *Ralstonia eutropha.* The host microorganism may be subjected to inactivation of the polyester synthase by a gene disruption treatment by means of a mutagen treatment or homologous recombination, and for example, a *Ralstonia eutropha* PHB-4 strain and the like may be used. The *Ralstonia eutropha* PHB-4 strain is available from the organization such as DSMZ.

The polyhydroxyalkanoic acid synthase gene used according to the present invention may be any one as long as it can accumulate P(3HB-co-3HH) among the genes derived from various PHA-accumulative organisms. Examples of such a gene include e.g., polyhydroxyalkanoic acid synthase genes isolated from *Aeromonas caviae* (Nonpatent Document 13), *Nocardia corallina* (GenBank Accession No. AF019964), and the like.
Further, as the polyhydroxyalkanoic acid synthase gene, any one having modification of a part of the base sequence of the gene effected such that the amino acid sequence is modified in the range not to abolish the intended enzyme activity can be also used. For example, a polyester synthase gene derived from *Aeromonas caviae* including substitution of the amino acid asparagine at position 149 with serine (N149S mutant gene), a polyester synthase gene derived from *Aeromonas caviae* including substitution of the amino acid aspartic acid at position 171 with glycine (D171G mutant gene) described in Nonpatent Document 16, a polyester synthase gene derived from *Aeromonas caviae* including combined substitution of the aforementioned two amino acids, or the like can be preferably used. The polyhydroxyalkanoic acid synthase gene used in the present invention is preferably a gene encoding an enzyme derived from *Aeromonas caviae,* or a mutant thereof, and is more preferably a gene encoding a mutant enzyme derived from *Aeromonas caviae* including substitution of the amino acid aspartic acid at position 171 with glycine,

The 3-ketoacyl-ACP reductase gene used in the present invention is a known gene *(fabG)* derived from an eucaryotic organism or a microorganism encoding 3-ketoacyl-ACP reductase (EC1.1.1.100), an enzyme in fatty acid biosynthetic pathway, Examples of the gene include e.g., a 3-ketoacyl-ACP reductase gene derived from *Escherichia coli* (Rawlings) et al., J. Biol. Chem. vol. 267, p 5751, (1992), DDBJ M84991) and the promoter sequence thereof (Podkovyrov et al., Nucleic Acids Res. vol. 24, p 1747, (1996), Zhang et al., J. Bacteriol. vol. 180, p 3295, (1998)), a 3-ketoacyl-ACP reductase gene derived from *Moraxella catarrhalis* (U. S. Patent No. 6632636), a 3-ketoacyl-ACP reductase gene derived from *Brassica napus* (U. S. Patent No. 6011201), a 3-ketoacyl-ACP reductase gene derived from *Staphylococcus aureus* (U. S. Patent No. 6110704), and the like, which can be used alone or in combination of more than one thereof. As the aforementioned 3-ketoacyl-ACP reductase gene, those derived from *Escherichia coli* are preferred.
The polyhydroxyalkanoic acid synthase gene and the 3-ketoacyl-ACP reductase gene described above may have an expression unit that functions in the host microorganism such as a promoter, a terminator and the like, in addition to the structure gene. In the plasmid for expression, one or more of the expression units may be present, and multiple units may be present,

In the present invention, the vector which can be used for introducing the gene into the host microorganism may be any known vector for microorganisms. For example, pBluescriptII, pUC series vectors and the like being vectors for *Escherichia coli,* as well as pJRD215 (ATCC 37533) and pBBR1 (Kovach et al., Biotechniques, 5: 800-802 (1994)) being wide host range vectors, and the like may be exemplified. Further, vectors produced by modifying these vectors can be also used. In particular, vectors produced by nonconjugal transferable modification of pJRD215 can be suitably used in light of safety. Examples of such modification include reduction or loss of the function by carrying out deletion, insertion, substitution or the like of a base in the region of *mob* gene and/or *orl*T sequence. Moreover, a vector miniaturized by deletion of an unnecessary region can be suitably used in light of improvement of transformation efficiency. pJRDdTc produced in Example herein can be suitably used as one example of the miniaturized vector produced by nonconjugal transferable modification of pJRD215.

The plasmid for expression having the polyhydroxyalkanoic acid synthase gene and the 3-ketoacyl-ACP reductase gene incorporated into a vector can be produced by any known method. The entire gene manipulation may be carried out as described in Molecular Cloning (Cold Spring Harbor Laboratory Press, (1989)). The enzyme, cloning host and the like used in the gene manipulation may be purchased from a supplier on the market, and can be used according to the protocol. The enzyme is not particularly limited as long as it is one which can be used in gene manipulation. Also, the cloning host is not particularly limited, but for example, an *Escherichia coli* strain DH5a and the like may be used.

The plasmid for expression having the polyhydroxyalkanoic acid synthase gene and the 3-ketoacyl-ACP reductase gene incorporated into a vector can be introduced into the host microorganism by any known method. For example, the electroporation method (Current Protocols in Molecular Biology, vol. 1, page 1.8.4, 1994), the calcium method (Lederberg et al., J. Bacteriol., 119: 1072-1074 (1974)) or the like can be employed.

As a preferred transformant of the present invention, for example, a transformant including the PHA synthase gene encoding a mutant enzyme derived from *Aeromonas caviae* having substitution of the amino acid aspartic acid at position 171 with glycine, and the 3-ketoacyl-ACP reductase gene derived from *Escherichia coli* introduced into a *Ralstonia eutropha* strain PHB-4 as a host may be exemplified.

By proliferation of thus produced transformant in the presence of a carbon source, the copolymerized polyester can be accumulated in the cells of the microorganism. As the carbon source, sugar, fat and oil, or fatty acid can be used. As the nutrient source other than the carbon source, a nitrogen source, an inorganic salt, or other organic nutrient source can be arbitrarily used.
Examples of the sugar include e.g., carbohydrate such as glucose and fructose, and the like. Examples of the fat and oil include fats and oils containing a saturated or unsaturated fatty acid having 10 or more carbon atoms in a large proportion, e.g., coconut oil, palm oil, palm kernel oil, and the like. Examples of the fatty acid include saturated or unsaturated fatty acids such as hexanoic acid, octanoic acid, decanoic acid, lauric acid, oleic acid, palmitic acid, linoleic acid, linolenic acid and myristic acid, or fatty acid derivatives such as esters and salts of these fatty acids, and the like.
Examples of the nitrogen source include e.g., ammonia, ammonium salts such as ammonium chloride, ammonium sulfate and ammonium phosphate, as well as peptone, meat extract, yeast extract, and the like.
Examples of the inorganic salt include e.g., potassium dihydrogen phosphate, dipotassium hydrogen phosphate, magnesium phosphate, magnesium sulfate, sodium chloride, and the like.
Examples of the other organic nutrient source include e.g., amino acids such as glycine, alanine, serine, threonine and proline; vitamins such as vitamin B1, vitamin B12 and vitamin C, and the like.

Culture temperature of the produced transformant may be a temperature at which the microorganism can grow, but is preferably from 20°C to 40°C. The culture time period is not particularly limited, but may be approximately from 1 to 10 days. The polyester accumulated in thus obtained culture bacterial cells can be collected by any known method.
For example, the following process may be employed. After the culture, the bacterial cells are separated from the culture medium by a centrifugal separator or the like, and the bacterial cells are washed with distilled water and methanol or the like, and dried. From the dried bacterial cells, the polyester is extracted using an organic solvent such as chloroform. Bacterial cell components are removed from this organic solvent solution including the polyester by filtration or the like, and a poor solvent such as methanol or hexane is added to the filtrate to permit precipitation of the polyester. Furthermore, the supernatant is removed by filtration or centrifugal separation, and dried. Accordingly, the polyester can be collected.
As a simplified process for verifying production of the polyester, a staining method with Nilered can be employed. More specifically, Nilered is added to an agar medium in which the recombinant bacterium is growing, and the recombinant bacterium is cultured for 1 to 7 days. Thus, observation of the presence of red coloration of the recombinant bacterium can verify the production of the polyester.
A method of producing a polyester using the transformed microorganism described above is also involved in the present invention.

### Effects of the Invention

According to the present invention, a transformed microorganism strain capable of highly producing PHA having an ultra high molecular weight that is excellent in characteristics to process into fiber and the like is provided, whereby simple and large-scale production of ultra high molecular weight PHA with high purity is enabled at a low cost.

### Best Mode for Carrying Out the Invention

Hereinafter, the present invention will be explained by way of Examples, but the present invention is not anyhow restricted by these Examples.

### (Example 1) Production of pJRDdTc vector

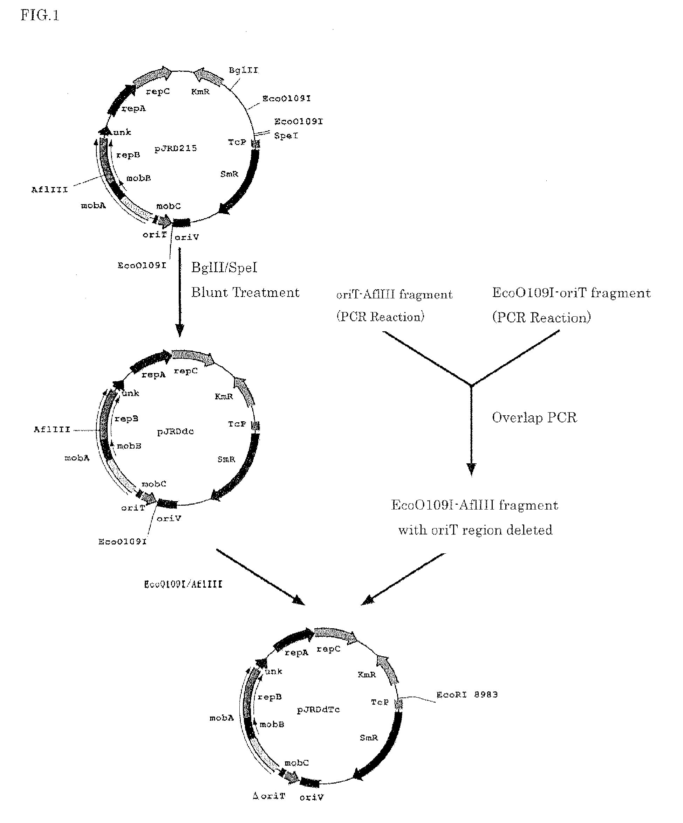

A vector pJRD215 (SEQ ID NO: 1) cleaved with restriction enzymes SpeI and BglII was blunt ended using a DNA Blunting Kit (manufactured by Takara Shuzo Co., Ltd.), and self-ligated. The product was referred to as pJRDdc (Fig. 1). The pJRDdc results from deletion of bases at position of from 8915 to 10055 including a cos region and the like, from pJRD215 set out in SEQ ID NO: 1.
Next, using primers set out in SEQ ID NO: 2 and SEQ ID NO: 3, a PCR was performed with pJRDdc as a template to obtain a DNA fragment of about 0.5 kb. Additionally, using primers set out in SEQ ID NO: 4 and SEQ ID NO: 5, a PCR was similarly performed with pJRDdc as a template to obtain a DNA fragment of about 2.4 kb. The overlap PCR method was performed utilizing the presence of an overlap in the resulting two fragments. Pyrobest (manufactured by Takara Shuzo Co., Ltd.) was used as a polymerase. The fragment of about 2.8 kb obtained by this overlap PCR method was cleaved with restriction enzymes EcoO109I and AflIII, and ligated to the aforementioned pJRDdc which had been similarly cleaved with EcoO109I and AflIII. Thus, pJRDdTc was obtained (see, Fig. 1). In other words, the vector pJRDdTc results from deletion of an *orlT* region (bases at position of from 3132 to 3169 in SEQ ID NO: 1) from pJRD215, and further deletion of the cos region (bases at position of from 8915 to 10055 in SEQ ID NO: 1) and the like.

### (Example 2) Construction of expression plasmid

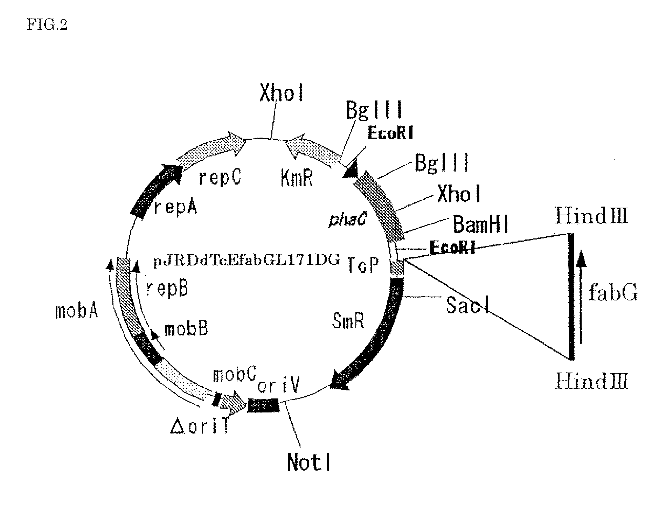

An expression plasmid for producing the ultra high molecular weight polyester was constructed as follows.
A fragment of a D171G mutant gene as the polyester synthase gene was produced by a PCR method. The D171G mutant gene has substitution of the amino acid aspartic acid at position 171 with glycine in the PHA synthase derived from *Aeromonas caviae* as set out in SEQ ID NO: 14. Therefore, the gene fragment EE32d13 derived from *Aeromonas caviae* described in Japanese Unexamined Patent Application Publication No. Hei 10-108682 was subcloned into the EcoRI site of pUC19 once, and PCR was performed using synthetic DNAs set out in SEQ ID NO: 6 and SEQ ID NO: 7 as a primer. The conditions included: (1) at 94°C for 2 min, (2) at 94°C for 30 sec, (3) at 55°C for 30 min, (4) at 72°C for 2 min, 25 cycles of the steps from (2) to (4), and (5) at 72°C for 5 min, and Ex Taq polymerase (manufactured by Takara Bio Inc.) was used as a polymerase. A D171G fragment was prepared by cleaving with a restriction enzyme EcoRI, and was inserted into the site which had been generated by cleaving with the same enzyme the pJRDdTc produced in Example 1, thereby producing pJRDdTc171DG. Next, an *Escherichia coli fabG* gene was amplified at its structural gene portion by performing PCR with the chromosomal gene of an *Escherichia* coli strain HB101 as a template using SEQ ID NO: 8 and 9 as a primer. The PCR conditions included (1) at 94°C for 2 min, (2) at 94°C for 30 sec, (3) at 45°C for 30 sec, (4) at 72°C for 1 min, 30 cycles of the steps from (2) to (4), and (5) at 72°C for 5 min, and La Taq polymerase (manufactured by Takara Bio Inc.) was used as a polymerase. Thereafter, the product was cleaved with SacI and BamHI. After annealing synthetic DNAs set out in SEQ ID NOs: 10 and 11 including a *fabG* gene terminator sequence (see, Rawlings et al., J. Biol. Chem., 267: 5751-5754 (1992)), it was cleaved with BglII and HindIII, and subcloned into HindIII site of pUC19 together with a *fabG* structural gene fragment which had been cleaved with SacI and BamHI. It was cleaved with XbaI and HindIII to prepare a fragment including the *fabG* gene, and subcloned into HindIII site of pUC19 together with a fragment obtained by annealing synthetic DNAs set out in SEQ ID NOs: 12 and 13 including a promoter sequence of a *fabG* gene (see, Podkovyrov et al., Nucleic Acids Res., 24: 1747-1752 (1996)) followed by cleaving with HindIII and NheI. A *fabG* gene fragment was prepared therefrom as a HindIII fragment, which was inserted into HindIII site of pJRDdTc171DG to accomplish an expression plasmid pJRDdTcEfabGL171DG (see, Fig. 2).

### Example 3) Production of transformant

(A transformant of a *Ralstonia eutropha* strain PHB-4 including this expression plasmid was produced with the electric pulse method. More specifically, a gene pulser manufactured by Bio-Rad Laboratories, Inc., was used as an apparatus for introducing the gene, and a cuvette having a gap of 0.2 cm also manufactured by Bio-Rad Laboratories, Inc., was used. To the cuvette were injected 400 µl of competent cells and 20 µl of the expression plasmid. The cuvette was set into the pulser, and the electric pulse was applied under a condition of a capacitance of 25 µF, a voltage of 1.5 kV, and a resistance of 800 Ω. After applying the pulse, the bacterial liquid in the cuvette was cultured with shaking in a Nutrient Broth medium (manufactured by DIFCO) at 30°C for 3 hrs, followed by culture in a selection plate (Nutrient Agar medium (manufactured by DIFCO), containing kanamycin 100 mg/L) at 30°C for 2 days to obtain the transformant.

### (Example 4) Selection of transformant

The transformant obtained in Example 3 was inoculated in a Nilered-containing medium (disodium hydrogen phosphate·12 hydrate 9 g, potassium dihydrogen phosphate 1.5 g, ammonium chloride 0.05 g, magnesium sulfate·7 hydrate 0.02 g, fructose 0.5 g, cobalt chloride·6 hydrate 0.25 ppm, iron (III) chloride·6 hydrate 16 ppm, calcium chloride·2 hydrate 10.3 ppm, nickel chloride·6 hydrate 0.12 ppm, copper sulfate·5 hydrate 0.16 ppm, Nilered 0.5 mg, and agar 15 g/L), and cultured at 30°C for 1 week, As a result, accumulation of the polyester in the bacterial cells was verified based on red coloration of the colony. The colony was selected, and used for production of the polyester.

### (Example 5) Production and purification of polyester

The composition of the seed medium was 1 w/v% Meat-extract, 1 w/v% Bacto-Trypton, 0.2 w/v% Yeast-extract, 0.9 w/v% Na₂HPO₄·12H₂O, and 0.15 w/v% KH₂PO₄ (pH 6.8).
The composition of the preculture medium was 1.1 w/v% Na₂HPO₄·12H₂O, 0.19 w/v% KH₂PO₄, 1.29 w/v% (NH₄)₂SO₄, 0.1 w/v% MgSO₄·7H₂O, 2.5 w/v% palm W olein oil, 0.5 v/v% trace metal salt solution (obtained by dissolving 1.6 w/v% FeCl₃·6H₂O, 1 w/v% CaCl₂·2H₂O, 0.02 w/v% CoCl₂·6H₂O, 0.016 w/v% CuSO₄·5H₂O and 0.012 w/v% NiCl₂·6H₂O in 0.1 N hydrochloric acid), and 5 x 10⁻⁶ w/v% kanamycin.
The composition of polyester-producing medium was 0.385 w/v% Na₂HPO₄·12H₂O, 0.067 w/v% KH₂PO₄, 0.291 w/v% (NH₄)₂SO₄, 0.1 w/v% MgSO₄·7H₂O, 0.5 v/v% trace metal salt solution (obtained by dissolving 1.6 w/v% FeCl₃·6H₂O, 1, w/v% CaCl₂·2H₂O, 0.02 w/v% CoCl₂·6H₂O, 0.016 w/v% CuSO₄·5H₂O and 0.012 w/v% NiCl₂·6H₂O in 0.1 N hydrochloric acid), 0.05 w/v% BIOSPUREX 200K (deforming agent: manufactured by Cognis Japan), and 5 x 10⁻⁶ w/v% kanamycin. With respect to the carbon source, a palm kernel oil olein that is a low-melting point fraction obtained from palm kernel oil by fractionation was used as a single carbon source, which was fed such that the specific substrate feeding rate became 0.08 to 0.1 (fat and oil (g)) x (net dry bacterial cell weight (g))⁻¹ x (h)⁻¹ throughout the culture.
The glycerol stock (50 µl) of the transformant obtained in Example 4 was inoculated into the seed medium (10 ml), and cultured for 24 hrs. The culture was inoculated at 1.0 v/v% into a 3-L jar fermenter (manufactured by B. E. MARUBISHI Co., Ltd., model MDL-300) charged with 1.8 L of the preculture medium. The operation conditions involved a culture temperature at 30°C, a rate of stirring of 500 rpm, and an aeration rate of 1.8 L/min. The culture was carried out for 28 hrs while adjusting the pH of from 6.7 to 6.8. For adjusting the pH, a 7% aqueous ammonium hydroxide solution was used.

For the polyester-producing culture, the preculture seed was inoculated at 5.0 v/v% into a 10-L jar fermenter (manufactured by B. E. MARUBISHI Co., Ltd., model MDL-1000) charged with 6 L of the producing medium. The operation conditions involved a culture temperature at 28 °C, a rate of stirring of 400 rpm, and an aeration rate of 3.6 L/min, with the pH adjusted to be from 6.7 to 6.8. For adjusting the pH, a 7% aqueous ammonium hydroxide solution was used. The culture was carried out for about 64 hrs, and after the completion of the culture, the bacterial cells were collected by centrifugal separation, washed with methanol, and freeze-dried. Measurement of the dry bacterial cell weight gave a value of 109.2 g/L
To about 1 g of the resulting dry bacterial cells was added 100 ml of chloroform, and the mixture was stirred at room temperature for about twenty-four hours. Then, the polyester in the bacterial cells was extracted. Following filtration of the bacterial cell residues, the filtrate was concentrated with an evaporator until the total volume of about 30 ml was attained. Thereafter, about 90 ml of hexane was gradually added thereto, and the mixture was left to stand while being gently stirred for 1 hour. The polyester separated out was collected by filtration, and was dried at 50°C for 3 hrs in vacuo. The weight of the dry polyester was measured, whereby the polyester content in the bacterial cells was determined. As a result, the polyester content in the transformant was such a high content as 68.6 (wt%) in 64 hrs.

### (Example 6) Analysis of 3HH composition (mol%) of polyester

Analysis of the 3HH composition of the polyester produced by the transformant was measured by gas chromatography as follows.
To about 20 mg of dry polyester were added 2 ml of a mixture of sulfuric acid-methanol (15:85) and 2 ml of chloroform. The mixture was enclosed airtight, and heated at 100°C for 140 min to obtain a methyl ester of the polyester degradation product. After cooling, thereto was added 1.5 g of sodium bicarbonate in small portions to effect neutralization, and the mixture was left to stand until generation of carbon dioxide gas ceased. After adding 4 ml of diisopropyl ether thereto followed by mixing well, the mixture was centrifuged, and the monomer unit composition of the polyester degradation product in the supernatant was analyzed by capillary gas chromatography. The gas chromatograph used was GC-17A manufactured by Shimadzu Corporation, and NEUTRA BOND-1 manufactured by GL Sciences, Inc., (column length: 25 m, column internal diameter: 0.25 mm, film thickness: 0.4 µm) was used as a capillary column. Helium was used as a carrier gas, and with a column inlet pressure of 100 kPa, the sample was injected in a volume of 1 µl. The temperature conditions involved an initial temperature of from 100 to 200°C at a rate of temperature elevation of 8°C/min, followed by additional temperature elevation of from 200 to 290°C at a rate of 30°C/min. As a result of the analysis under the conditions as described above, the polyester had the 3HH composition of 4.0 (mol%) at the end of culture for 64 hrs.

### (Example 7) Analysis of weight average molecular weight (Mw) of polyester

Analysis of the weight average molecular weight (Mw) of the polyester was carried out by the gel permeation chromatography method. The extracted polyester in an amount of about 5 mg was dissolved in 10 ml of chloroform, and the solution was filtrated with a 0.2 µm filter to give a sample for the measurement. Thus, a 0,05 ml aliquot of the sample was used for the analysis.
The measurement system was SLC-10A (manufactured by Shimadzu Corporation), with two columns Shodex GPC K-806L (manufactured by Showa Denko K. K.) serially connected, and the measurement was conducted at 40°C. The mobile phase was chloroform at a rate of 1.0 ml/min, and an RI detector (RID-10A, manufactured by Shimadzu Corporation) was used. As standards, similarly treated polystyrene (manufactured by Showa Denko K. K., weight average molecular weight: about 7,000,000, about 1,070,000, 150,000, 30,000) was used, and the weight average molecular weight of the polyester was determined using the calibration curve. As a result, the polyester produced by the transformant had a weight average molecular weight (Mw) of about 5,100,000, falling within the ultra high molecular weight.

### Industrial Applicability

According to the present invention, a transformed microorganism strain capable of highly producing PHA having an ultra high molecular weight that is excellent in characteristics to process into fiber and the like is provided, whereby simple and large-scale production of ultra high molecular weight PHA with high purity is enabled at a low cost.

### BRIEF DESCRIPTION OF THE DRAWINGS

[Fig. 1] A schematic drawing illustrating the production steps of a pJRDdTc vector constructed in Example 1.
[Fig. 2] A schematic drawing illustrating the constitution of an expression plasmid pJRDdTcEfabGL171DG constructed in Example 2.

## Claims

1. A microorganism which is obtained by introducing a polyhydroxyalkanoic acid synthase gene and a 3-ketoacyl-ACP reductase gene into at least one host microorganism selected from the group consisting of those belonging to genus *Ralstonia,* genus *Aeromonas,* genus *Alcaligenes* and genus *Pseudomonas* to allow for transformation, and which is capable of accumulating a copolymerized polyester composed of monomer units of 3-hydroxybutyric acid and 3-hydroxyhexanoic acid.

2. The microorganism according to claim 1 wherein the host microorganism is *Ralstonia eutropha.*

3. The microorganism according to claim 1 or 2 wherein the 3-ketoacyl-ACP reductase gene is a gene derived from *Escherichia coli.*

4. The microorganism according to any one of claims 1 to 3 wherein the polyhydroxyalkanoic acid synthase gene is a gene encoding an enzyme derived from *Aeromonas caviae,* or a mutant thereof.

5. The microorganism according to claim 4 wherein the mutant comprises at least one amino acid substitution of either one of the following (a) or (b):
(a) substitution of the amino acid asparagine at position 149 with serine; or
(b) substitution of the amino acid aspartic acid at position 171 with glycine.

6. The microorganism according to any one of claims 1 to 3 wherein the polyhydroxyalkanoic acid synthase gene encodes a mutant enzyme derived from *Aeromonas caviae* comprising substitution of the amino acid aspartic acid at position 171 with glycine.

7. A method of producing a polyester using the microorganism according to any one of claims 1 to 6.
